(19) Europäisches Patentamt

European Patent Office

Office européen des brevets

(11) EP 1 308 522 A1

(12) EUROPEAN PATENT APPLICATION

(43) Date of publication:
07.05.2003 Bulletin 2003/19

(51) Int Cl.$^7$: C12Q 1/68

(21) Application number: 01126244.1

(22) Date of filing: 05.11.2001

(84) Designated Contracting States:
AT BE CH CY DE DK ES FI FR GB GR IE IT LI LU MC NL PT SE TR
Designated Extension States:
AL LT LV MK RO SI

(71) Applicant: Haferlach, Torsten, PD Dr. Dr.
81477 München (DE)

(72) Inventors:
• Haferlach, Torsten
  81477 München (DE)
• Schoch, Claudia
  81477 München (DE)
• Kohlmann, Alexander
  92318 Neumarkt (DE)

• Schnittger, Susanne
  81375 München (DE)
• Dugas, Martin
  81377 München (DE)
• Kern, Wolfgang
  82319 Starnberg (DE)
• Eils, Roland
  69198 Schriesheim (DE)
• Brors, Benedikt
  69221 Dossenheim (DE)
• Mergenthaler, Susanne
  82131 Gauting (DE)

(74) Representative: Schüssler, Andrea, Dr.
Kanzlei Huber & Schüssler
Truderinger Strasse 246
81825 München (DE)

(54) Novel genetic markers for leukemias

(57) The present invention relates to a diagnostic composition comprising nucleic acid molecules which are capable of specifically hybridizing to the mRNAs of genes showing abnormal gene expression associated with leukemia, preferably AML, CML, ALL and CLL. The present invention also relates to the use of said nucleic acid molecules for diagnosis of further genetically defined leukemia subtypes or a disposition to a leukemia.

EP 1 308 522 A1

**Description**

The present invention relates to a diagnostic composition comprising nucleic acid molecules which are capable of specifically hybridizing to the mRNAs of genes showing abnormal gene expression associated with leukemias. The present invention also relates to the use of said nucleic acid molecules for diagnosis of a leukemia or leukemia subtypes or a disposition to a leukemia.

Today leukemias are classified into four different groups: acute myeloid (AML), acute lymphatic (ALL), chronic myeloid (CML) and chronic lymphatic leukemia (CLL): Within these groups, several subcategories can be identified further using a panel of standard techniques as described below. The incidence of leukemias is increasing with age and is 5/100.000/year in AML, 1/100.000/year in ALL, 1/100.000 in CML and 6/100.000/year in CLL. Several methods for classification have to be applied at diagnosis and before treatment starts: cytomorphology and cytochemistry, multiparameter-immunophenotyping, cytogenetics including fluorescence in situ hybridization, and molecular techniques such as polymerase chain reaction (PCR). So far only a combination of these techniques allows a precise diagnosis which is necessary to apply state of the art treatment. As the exact diagnosis is mandatory for example in CML the detection of a specific cytogenetic abnormality, the translocation (9;22) or its molecular counterpart, the BCR/ABL rearrangement is required to establish the diagnosis of CML. While all patients with CML show a BCR-ABL-rearrangement and are therefore homogenous with regard to the primary genetic abnormality, in AML and ALL at least 10-15 different subgroups have been identified on the morphological, genetical or molecular level. Also in CLL several subgroups can be clearly separated. These different subcategories in leukemias are associated with varying clinical outcome and therefore are the basis for different treatment strategies. The importance of highly specific classification may be illustrated in detail further for the AML as a very heterogeneous group of diseases.

Data from clinical trials showed that outcome of patients with AML differs in a broad range. Several parameters influencing prognosis have been identified. These can be assigned to different categories: patients characteristics (i. e. age, comorbidity), therapy, and biology of the AML. Therefore, a lot of effort was invested to identify biological entities and to distinguish subgroups of AML which are associated with a favorable, intermediate or unfavorable prognosis, respectively. In order to allow a comparison between different studies a classification of AML was mandatory. In 1976 the FAB-classification was proposed by the French-American-British cooperative group which was based on cytomorphology and cytochemistry in order to separate AML subgroups according to the morphological appearance of blasts in the blood and bone marrow. In addition, it was recognized that genetic abnormalities occurring in the leukemic blast had a major impact on the morphological picture and even more on the prognosis. So far, the karyotype of the leukemic blasts is the most important independent prognostic factor regarding response to therapy as well as survival. For clinical purposes karyotype analysis allows to discriminate between three major prognostic groups. A favorable outcome under currently used treatment regimens with cure rates from 50% up to 85% was observed in several studies in patients with a) t(8;21) (q22;q22) occuring in AML M2, b) inv(16) (p13q22) occurring in AML M4eo and c) t(15;17) (q22;q11-12) occurring in AML M3/M3v. In contrast, chromosome aberrations with an unfavorable clinical course are -5/del(5q), -7/del (7q), inv(3)/t(3;3) and complex aberrant karyotypes with cure rates of only 10%. The remainder AML patients are assigned to a prognostically intermediate group. This latter group is very heterogeneous because it includes patients with a normal karyotype as well as those with rare chromosome aberrations with yet unknown prognostic impact.

The subclassification of leukemias becomes increasingly important to guide therapy. Thus, the development of new, specific treatment approaches requires the identification of specific subtypes that may benefit from a distinct therapeutic protocol. It has already been shown in two entities that the development of specific drugs can improve outcome of distinct subsets of leukemia. One important example is the development of a new therapeutic drug (STI571) for the treatment of chronic myeloid leukemia (CML): this designed molecule inhibits the CML specific chimeric tyrosine kinase BCR-ABL generated from the genetic defect observed in CML, the BCR-ABL-rearrangement due to the translocation between chromosomes 9 and 22 (t(9;22)(q34;q11)). First data show that therapy response is dramatically higher in patients treated with this new drug as compared to all other drugs that had been used so far. Another example is the subtype of acute myeloid leukemia AML M3 and its variant M3v both with karyotype t(15;17)(q22;qll-12). The introduction of a new drug (all-trans retinoic acid - ATRA) has improved the outcome in this subgroup of patient from about 50% to 85% long-term survivors. As it is mandatory for these patients suffering from these specific leukemia subtypes to be identified as fast as possible so that the best therapy can be applied, diagnostics today must accomplish subclassification with maximal precision. Not only for these subtypes but also for several other leukemia subtypes different treatment approaches could improve outcome. Therefore, rapid and precise identification of distinct leukemia subtypes is the future goal for diagnostics.

So far a combination of methods is necessary to obtain the most important information in leukemia diagnostics: Analysis of the morphology and cytochemistry of bone marrow blasts and peripheral blood cells is necessary to establish the diagnosis. In some cases the addition of immunophenotyping is mandatory to separate very undifferentiated AML from acute lymphoblastic leukemia and CLL. Leukemia subtypes investigated can be diagnosed by cytomorphology alone, if an expert reviews the smears. However, a genetic analysis based on chromosome analysis, fluorescence in situ hybridization or RT-PCR and immunophenotyping is required in order to assign all cases in to the right category. The aim of these techniques besides diagnosis is mainly to determine the prognosis of the leukemia. A major disadvantage of these methods, however, is that viable cells are necessary as the cells for genetic analysis have to divide in vitro in order to obtain metaphases for the analysis. Another problem is the long time of 72 hours from receipt of the material in the laboratory to obtain the result. Furthermore, great experience in preparation of chromosomes and even more in analyzing the karyotypes is required to obtain the correct result in at least 90% of cases. These experts in their field are necessary for all other techniques mentioned above as well.

Thus, the technical problem underlying the present invention is to provid means for leukemia diagnostics which overcome the disadvantages of the prior art diagnostic methods.

The solution to said technical problem is achieved by providing the embodiments characterized in the claims. Based on biomathematical analysis of gene expression profiles a subset of genes could be introduced which builds up the basis for designing and developing a novel diagnostic approach based on microarray technology which abolishes todays standard procedures in diagnosis of leukemia. These standard diagnostic procedures require more and more centralized core facilities with both personal experts in the fields of cytomorphology, cytogenetics and molecular genetics and expensive lab equipment, which causes increasing costs for adaequate diagnosis. The present invention provides a novel cost-effective diagnostic tool, which is less time consuming, easy to operate but nevertheless as accurate and save as all standard methods combined today. The set of genes allows to assign clinical samples either as healthy or malignant simply based on their gene expression profiles. This is the basis for such a diagnostic tool. Furthermore, these genes already allow to predict the diagnoses based on the genetic abnormality of the expression pattern and to discriminate between different prognostic relevant entities.

The development of a leukemia diagnostic tool, preferably microarray based, allows for all patients and specimen a reproducible, highly specific and rapid method to obtain important information for treatment strategies in leukemia. This technique can be established in every laboratory using basic methods of molecular biology and does not require hematologists or cytogeneticists with several years of experience in leukemia diagnostics. Material for the analysis can be sent over large distances as it is not necessary that cells arrive viable in the laboratory. Therefore, a centralization of leukemia diagnostics with very high quality is possible.

Moreover, the accumulation of an immense knowledge about gene expression profiles in leukemia subtypes, which are not characterized by specific genetic abnormalities, leads to a more precise classification compared to all other methods used so far. In addition, these data are helpful for the understanding of the pathogenesis of leukemia and will allow to identify genes which are specifically dysregulated. They may be considered as potential targets for therapeutic interventions specifically designed for the different leukemia subtypes.

Accordingly, the present invention relates to a diagnostic composition comprising at least one nucleic acid molecule, preferably (a) single-stranded nucleic acid molecule(s), which is capable of specifically hybridizing to the mRNA of at least one gene listed in Table 1.

The use of said nucleic acid molecules for diagnosis of leukemia subtypes, preferably based on microarray technology, offers the following advantages: (1) more rapid and more precise diagnosis, (2) easy to use in laboratories without specialized experience, (3) abolishes the requirement for analyzing viable cells for chromosome analysis (transport problem), (4) very experienced hematologists for cytomorphology and cytochemistry, immunophenotyping as well as cytogeneticists and molecularbiologists are no longer required, and (5) improves the subclassification of leukemia due to the definition of new entities based on gene expression profiles in those subtypes that are not clearly defined with the methods of the prior art (class discovery).

As used herein, the term "capable of specifically hybridizing" has the meaning of hybridization under conventional hybridization conditions, preferably under stringent conditions as described, for example, in Sambrook et al., Molecular Cloning, A Laboratory Manual, 2nd edition (1989) Cold Spring Harbor Laboratory Press, Cold Spring Harbor, NY. Also contemplated are nucleic acid molecules that hybridize at lower stringency hybridization conditions. Changes in the stringency of hybridization and signal detection are primarily accomplished through the manipulation of formamide concentration (lower percentages of formamide result in lowered stringency), salt conditions, or temperature. For example, lower stringency conditions include an overnight incubation at 37øC in a solution comprising 6X SSPE (20X SSPE = 3M NaCl; 0.2M NaH2PO4; 0.02M EDTA, pH 7.4), 0.5% SDS, 30% formamide, 100 æg/ml salmon sperm blocking DNA, followed by washes at 50øC with 1 X SSPE, 0.1% SDS. In addition, to achieve even lower stringency, washes performed following stringent hybridization can be done at higher salt concentrations (e.g. 5X SSC). Variations in the above conditions may be accomplished through the inclusion and/or substitution of alternate blocking reagents used to suppress background in hybridization experiments. The inclusion of specific blocking reagents may require modification of the hybridization conditions described above, due to problems with compatibility.

As a hybridization probe (or primer) nucleic acid molecules can be used, for example, that have exactly or basically the nucleotide sequence of the genes depicted in Table 1 or parts of these sequences. The term nucleic acid molecule as used herein also comprises fragments which are understood to be parts of the nucleic acid molecules that are long enough to specifically hybridize to transcripts of the genes of Table 1. These nucleic acid molecules can be used, for example, as probes or primers in a diagnostic assay. Preferably, the nucleic acid molecules of the present invention have a length of at least 13, in particular of at least 20 and particular preferred of at least 25 nucleotides. The nucleic acid molecules of the invention or parts therefrom can also be used, for example, as primers for a PCR reaction. The fragments used as hybridization probe can be synthetic fragments that were produced by means of conventional synthesis methods.

In a preferred embodiment, the diagnostic composition of the present invention comprises at least nucleic acid molecules which are capable of specifically hybridizing to the mRNAs of at least genes listed in Table 1.

In a more preferred embodiment, the diagnostic composition of the present invention comprises at least nucleic acid molecules which are capable of specifically hybridizing to the mRNAs of at least genes listed in Table 1. In the most preferred embodiment, the diagnostic composition of the present invention comprises at least nucleic acid molecules which are capable of specifically hybridizing to the mRNAs of at least all genes listed in Table 1.

In a further preferred embodiment, the nucleic acid molecules of the diagnostic composition of the present invention are bound to (a) a solid support, for example, a polystyrene microtiter dish or nitrocellulose membrane or glass surface or (b) to non-immobilised particles in solution.

In an even more preferred embodiment, the nucleic acid molecules of the diagnostic composition are present in a microarray format which can be established according to well known methods; for details see ,e.g., [http://www.affymetrix.com/technology/tech_spotted.html; http: //www.affymetrix.com/technology/tech_probe.html]

The present invention also provides the use of (a) nucleic acid molecule(s) of the present invention for the preparation of a diagnostic composition for the diagnosis of a leukemia or for the diagnosis of several subtypes or a disposition to a leukemia. For the diagnosis of a particular leukemia subtype, preferably, at least 5 different nucleic acid molecules are used as probes. For diagnosis, preferably, bone marrow or peripheral blood can be used. For diagnosis, the target sample is contacted with a (a) nucleic acid molecule(s) of the present invention and the concentration of individual mRNAs is compared with the mRNA expression profile levels of a test sample obtained from healthy donors.

The nucleic acid molecule is typically a nucleic acid probe for hybridization or a primer for PCR. The person skilled in the art is in a position to design suitable nucleic acids probes based on the information provided in Table 1.

The target cellular component, i.e. mRNA, e.g., in bone marrow or blood (BM), may be detected directly in situ, e.g. by in situ hybridization or it may be isolated from other cell components by common methods known to those skilled in the art before contacting with a probe. Detection methods include Northern blot analysis, RNase protection, in situ methods, e.g. in situ hybridization, in vitro amplification methods (PCR, LCR, QRNA replicase or RNA-transcription/amplification (TAS, 3SR), reverse dot blot disclosed in EP-B1 0 237 362))and other detection assays that are known to those skilled in the art. Preferably, detection is based on a microarray.

Products obtained by in vitro amplification can be detected according to established methods, e.g. by separating the products on agarose gels and by subsequent staining with ethidium bromide. Alternatively, the amplified products can be detected by using labeled primers for amplification or labeled dNTPs.

The probes can be detectably labeled, for example, with a radioisotope, a bioluminescent compound, a chemiluminescent compound, a fluorescent compound, a metal chelate, biotin or an enzyme.

**Brief Description of the Drawings:**

| | |
|---|---|
| Figure 1a: | Principal Component Analysis |
| Figure 1b: | Hierarchical Cluster Analysis |
| Figure 2: | Classification Accuracy |
| Figures 3a, 3b1, 3b2: | PCA of AML data based on 312 genes |
| Figure 4: | Decision Trees according to I(E) |
| Figure 5a: | Pair-wise Comparison of Normal BM and AML |
| Figure 5b: | Principal Component Analysis |
| Figure 5c: | Hierarchical Cluster Analysis |
| Figure 6a: | Pair-wise Comparison of Normal BM and ALL |
| Figure 6b: | Principal Component Analysis |
| Figure 6c: | Hierarchical Cluster Analysis |
| Figure 7a: | Pair-wise Comparison of Normal BM and CML |
| Figure 7b: | Principal Component Analysis |
| Figure 7c: | Hierarchical Cluster Analysis |
| Figure 8a: | Pair-wise Comparison of Normal BM and CLL |
| Figure 8b: | Principal Component Analysis |

Figure 8c: Hierarchical Cluster Analysis
Figure 9a: AML-WHO Classification
Figure 9b: Principal Component Analysis
Figure 9c: Hierarchical Cluster Analysis
Figure 10a: Comparison of Normal BM versus Leukemia
Figure 10b: Principal Component Analysis
Figure 10c: Hierarchical Cluster Analysis

The following Examples illustrate the invention.

## EXAMPLE 1

### General methods

(A) Selection and characterization of leukemia samples

Bone marrow (BM) aspirates were taken at the time of the initial diagnostic biopsy and remaining material was immediately lysed in RLT buffer (Qiagen), frozen and stored at -80 C until preparation for gene expression analysis. For microarray analysis the GeneChip System (Affymetrix, Santa Clara, CA, USA) was used. The targets for GeneChip analysis were prepared according to the current Expression Analysis Technical Manual. Briefly, frozen lysates of the leukemia samples were thawed, homogenized (QIAshredder, Qiagen) and total RNA extracted (RNeasy Mini Kit, Qiagen). Normally 10 μg total RNA isolated from 1 x 10$^7$ cells was used as starting material in the subsequent cDNA-Synthesis using Oligo-dT-T7-Promotor Primer (cDNA synthesis Kit, Roche Molecular Biochemicals). The cDNA was purified by phenol-chlorophorm extraction and precipitated with 100% Ethanol over night. For detection of the hybridized target nucleic acid biotin-labeled ribonucleotides were incorporated during the in vitro transcription reaction (Enzo® BioArray™ HighYield™ RNA Transcript Labeling Kit, ENZO). After quantification of the purified cRNA (RNeasy Mini Kit, Qiagen), 15 ug were fragmented by alkaline treatment (200 mM Tris-acetate, pH 8.2, 500 mM potassium acetate, 150 mM magnesium acetate) and added to the hybridization cocktail sufficient for 5 hybridizations on standard Gene-Chip microarrays. Before expression profiling "Test3 Probe Arrays" (Affymetrix) were choosen for monitoring of the integrity of the cRNA. Only labeled cRNA-cocktails which showed a ratio of the messured intensity of the 3' to the 5' end of the GAPDH gene less than 3.0 were selected for subsequent hybridization on HG-U95Av2 probe arrays (Affymetrix). Washing and staining of the probe arrays was performed as described by Lipshutz et al., Nat.Genet. 21 (1. Suppl.)(1999), 20-4; Lockhart et al., Nat.Biotechnol. 14(13) (1996), 1675-1680). The Affymetrix software (Microarray Suite, Version 4.0.1) extracted fluorescence intensities from each element on the arrays as detected by confocal laser scanning according to the manufacturers recommendations.

(B) Data analysis

Class separation by principal component analysis and hierarchical cluster analysis: In a first step, the dimensionality of the number of genes was reduced. Therefore, the data were scaled from each array to a target intensity value 50 (Affymetrix Microarray Suite) in order to be able to perform inter-array comparisons. Then all data was analyzed using Significance Analysis of Microarrays (Multiclass Response, Stanford University) (Tusher et al., PNAS USA 98 (9), (2001), 5116-5121) and a distinct number of genes based on a permutations test was selected. This reduced set of genes which showed to be significant then was analyzed using the Java application J-Express analysis tool (www.molmine.com), Dysvik and Jonassen, Bioinformatics 17(4) (2001), 369-370). Principal Component Analysis and Hierarchical Cluster Analysis (parameters Cluster method: single linkage and Distance metric: euclidean) showed a clear separation of analyzed groups of samples e.g. healthy bone marrow versus leukemia.

(C) Identification of differentially expressed genes according to Golub et al. (Science 286(5439) (1999), 531-537)

A previously described method (Golub et al., Science 286(5439) (1999), 531-537) was modified in order to reduce the number of candidate genes that could distinguish between our leukemic samples of interest. In a first step the raw data was scaled using Affymetrix software (target intensity 50 for all genes).

To avoid division by zero or negative numbers as occuring due to the current expression algorithm (Affymetrix) all average intensities were set of 20 or less to 20. Briefly, for a more detailed gene expression profiling the data analysis method according to Golub et al. using weighted voting was applied. In a first step, gene expression levels were log-transformed with a cut-off value set at 20 units. To assess the significance of selected genes a leave-one-out cross-validation was performed. Only those genes were considered important which were contained in all cross validation classificators. To determine the association between genes by chance a permutation test (100 cycles) was performed. Because the number of informative genes, which are able to discriminate between samples, is unknown, the Golub method was applied for different numbers of informative genes (range: 10-200). The minimal set of genes which provided optimal classification accuracy was selected to avoid overfitting.

**Example 2**

**Identification of genes, the aberrant expression of which is associated with a particular leukemia subtype**

Monitoring the gene expression level of thousands of mRNA transcripts simultaneously in one experiment is the key technology to find out the specific genes which allow the subsequent development of a class prediction model. Thus, the Affymetrix (Santa Clara, USA) oligonucleotide microarray technology (GeneChip© Instrument System) was used to obtain gene expression profiles of each individual clinical sample of interest. The HG-U95Av2 probe arrays gave information about the relative mRNA abundance of about 12,000 full length human genes which are represented on these high-density oligonucleotide microarrays.

In total, 8 bone marrow samples of healthy volunteers and leukemia patients were investigated. Five different types of bioinformatic calculations were performed:

(I) Three distinct genetic subtypes of AML

Three defined cytogenetic aberrations t(8;21)(q22;q22) (n=9), t(15;17) (q22;q12) (n=18) and M4eo with inv (16) (p13q22) (n=10) were selected corresponding to the 4 FAB-subtypes AML M2, M3 or M3v and M4eo, respectively. After bone marrow aspirates from 37 untreated patients with newly diagnosed AML were obtained, all cases were characterized by cytomorphology, cytogenetics and by molecular genetics. AML subtypes M3 and M3v both carry the same chromosomal aberration but differ in morphological aspects like nuclear configuration, granulation and clinical aspects like white blood cell count (WBC), respectively. In all cases, these balanced abnormalities were confirmed by fluorescence in-situ hybridization. The corresponding fusion transcript was detected by RT-PCR and/or quantitative real time PCR. The median age of the patients was 53 years (range, 19-82 years) and did not differ between the respective groups. The median WBC count was 17.0 G/l (range, 0.8-168.0 G/l) and was strikingly lower in patients with AML M3 as compared to all other patients.

Methods used:

(A) Selection and characterisation of leukemia samples

Bone marrow (BM) aspirates from 37 AML patients standing for four morphological and three underlying cytogenetic subgroups were obtained that were sent to the Laboratory of Leukemia Diagnostics (LFL) for central diagnosis (Klinikum Grosshadern, Munich, Germany). They were selected for this study on the basis of several criteria. It was mandatory that none of the patients had been treated. All samples, exclusively newly diagnosed in our laboratory, had to be well characterized as de novo AML and diagnosis had been proven by cytomorphology, cytogenetics, flow cytometry and molecular genetics in every single case. All samples for gene expression analysis were taken at the time of the initial diagnostic biopsy when remaining material was immediately lysed in RLT buffer (Qiagen), frozen and stored at -80° C until preparation for gene expression analysis.

(B) Microarray experiments

For microarray analysis the GeneChip System (Affymetrix, Santa Clara,CA, USA) was used. The targets for GeneChip analysis were prepared according to the current Expression Analysis Technical Manual. Briefly, frozen lysates of the leukemia samples were thawed, homogenized(QIAshredder, Qiagen) and total RNA extracted (RNeasy Mini Kit, Qiagen). Normally 10 μg total RNA isolated from $1 \times 10^7$ cells was used as starting material in the subsequent cDNA-Synthesis using Oligo-dT-T7-Promotor Primer (cDNA synthesis Kit, Roche Molecular Biochemicals). The cDNA was purified by phenol-chlorophorm extraction and precipitated with 100% Ethanol over night. For detection of the hybridized target nucleic acid biotin-labeled ribonucleotides were incorporated during the in vitro transcription reaction (Enzo® BioArray™ HighYield™ RNA Transcript Labeling Kit, ENZO). After quantification of the purified cRNA (RNeasy Mini Kit, Qiagen), 15 μg were fragmented by alkaline treatment (200 mM Tris-acetate, pH 8.2, 500 mM potassium acetate, 150 mM magnesium acetate) and added to the hybridization cocktail sufficient for 5 hybridizations on standard GeneChip microarrays. Before expression profiling "Test3 Probe Arrays" (Affymetrix) were choosen for monitoring of the integrity of the cRNA. Only labeled cRNA-cocktails which showed a ratio of the measured intensity of the 3' to the 5' end of the GAPDH gene less than 3 were selected for hybridization on HG-U95Av2 probe arrays (Affymetrix). Washing and staining the probe arrays was performed as described. The Affymetrix software (Microarray Suite, Version 4.0.1) extracted fluorescence intensities from each element on the arrays as detected by confocal laser scanning according to the manufacturers recommendations.

(C) Class separation by principal component analysis and hierarchical cluster analysis

In a first step, the dimensionality of the number of genes was reduced. Therefore, the data from each array were scaled to a target intensity value 50 (Affymetrix Microarray Suite) in order to be able to perform inter-array comparisons. Then all data was analyzed using Significance Analysis of Microarrays (Multiclass Response, Stanford University) and 580 genes were selecetd based on a permutations test. This reduced set of genes which showed to be significant then was analyzed using the Java application J-Express analysis tool. Principal Component Analysis and Hierarchical Cluster Analysis (parameters Cluster method: single linkage and Distance metric: euclidean) showed a clear separation of analyzed groups of samples e.g. healthy bone marrow versus leukemia.

(D) Identification of differentially expressed genes according to Golub

This analysis was carried out as described in Example 1 (C), above. Briefly, classification of tumor samples was achieved by using a set of samples whose class had been already determined. This set was called *training set*. By using the oligonucleotide microarrays (Lockhart et al., Nat. Biotechnol. 14 (1996), 1675-1680), the transcript levels in training set samples were measured for those genes that were represented on the microarray. The values for "transcription strength" were determined by averaging the values of a set of probes which were compared to a set of nearly identical probes containing a single mismatch. This was performed by using methods provided by the oligonucleotide array of Affymetrix Inc.

(E) Principle Components Analysis, Classifier and Decisions Trees

In order to obtain comparable values between different samples, they had to be standardised first. The method followed that described in Beißbarth et al. (Nat. Biotechnol. 14 (1996), 1675-1680), except that correcting for (additive) background had been omitted. In brief, the data from one of the samples were declared to serve as a "standard", and the values from all other samples were adapted to this standard. For every possible comparison to this standard, a set of "reliable" values was determined by calculating the correlation coefficient for a series of intervals of increasing length. The lower bound of reliability was the bound of the interval that had a correlation coefficient less than or equal to the smaller intervals. From all reliable values, a (logarithmised) correction factor was calculated by computing the median of the differences of the logarithmic values. Values that were zero or negative prior to taking the logarithm were not taken into account.

The obtained data matrix contained values from one sample per column. The gene expression profile across all samples for one gene or gene fragment represented on the oligonucleotide microarray was contained in a row of the matrix. To allow for rapid calculation of the classifier and to reduce memory usage, certain genes were pre-selected from the set of all genes represented on the array. The following criteria were applied:

$$\sum_{i=1}^{k}\left|\mu_i - \overline{\mu}\right| - \sum_{i=1}^{k}\sigma_i > 0 \quad (1)$$

$$\frac{\sum_{i=1}^{k}\left|\mu_i - \overline{\mu}\right|}{\sum_{i=1}^{k}\sigma_i} > t \quad (2)$$

$\mu_i$ refers to the average of the i-th class ($i$=l,...,k), $\mu$ to the total average, $\sigma_i$ to the standard deviation of the i-th class and $t$ to an arbitrary treshold $\leq 1$. Selection by these methods resulted typically in a reduction in the number of genes by a factor of 10-30. To check the quality of the selection procedure, the first two principal components (Jolliffe, Principle Components Analysis (1986), Springer (New York)) for the samples were plotted. This allowed to judge whether or not a rigorous discrimination was possible between the different classes.

For construction of the classifier, decision trees (Breiman et al., Classification and regression trees, Wadsworth & Brooks/Cole (Monterey)) were used. Simple decision trees that discriminate between *n* classes by using only transcription levels for (*n*-1) genes were used. They were trained and the selected genes were the discarded from the original data set. A new tree was constructed by using the truncated data set and the entire procedure was iterated until a predetermined number of trees was reached. The optimal number of trees could be estimated by counting the number of misclassifications of classifiers built from different numbers of trees. For this, an independent data set of cross-validation had to be used. The final vote of the multi-classifier was obtained by applying a vote-by-majority rule to the predictions of the contained trees. In the example of the present invention 15 decision trees had been used for the multi-classifier. This allowed perfect classification of 100% of the samples, discriminating between classes that were given by chromosomal aberrations. To estimate generalisation properties, i.e. how accurate the classifier may perform on samples that have not been used for training, cross-validation had been used (Efron and Tibshirani, An introduction to the bootstrap (1993), Chapman & Hall (New York, London), pp. 237-247).

Results (Golub Method)

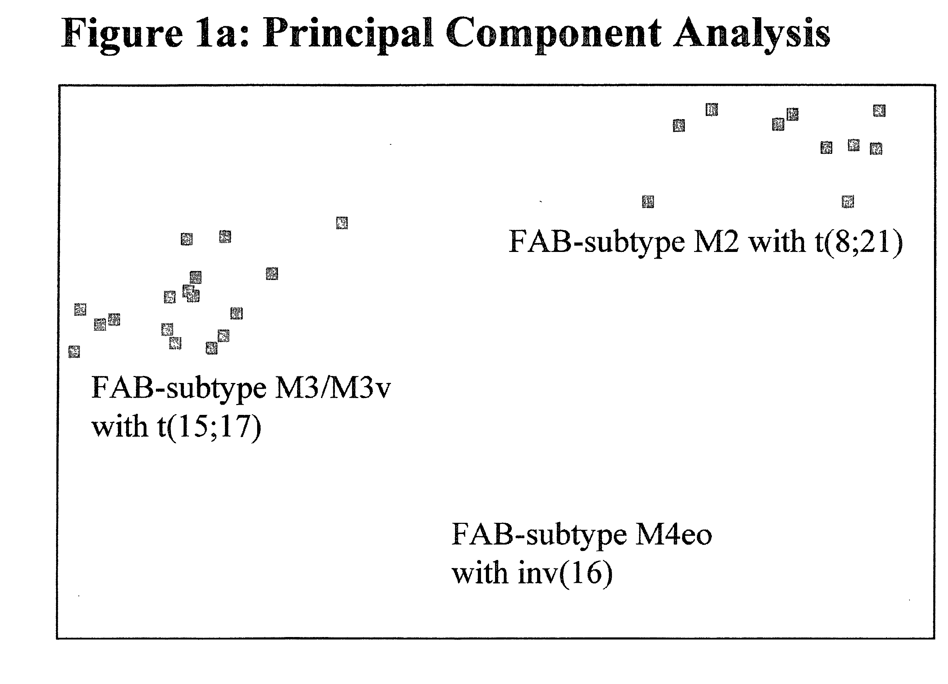

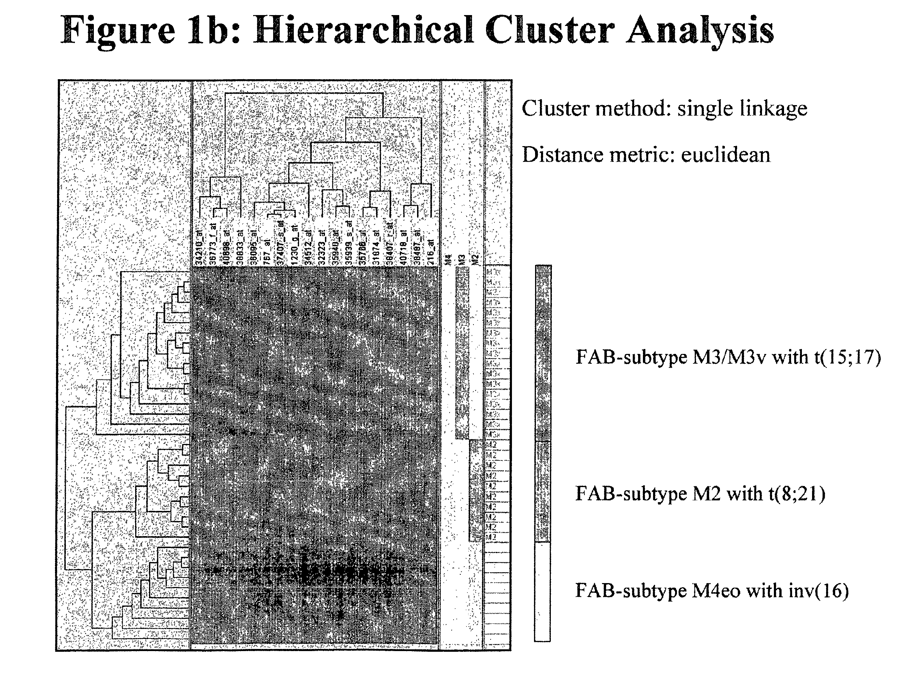

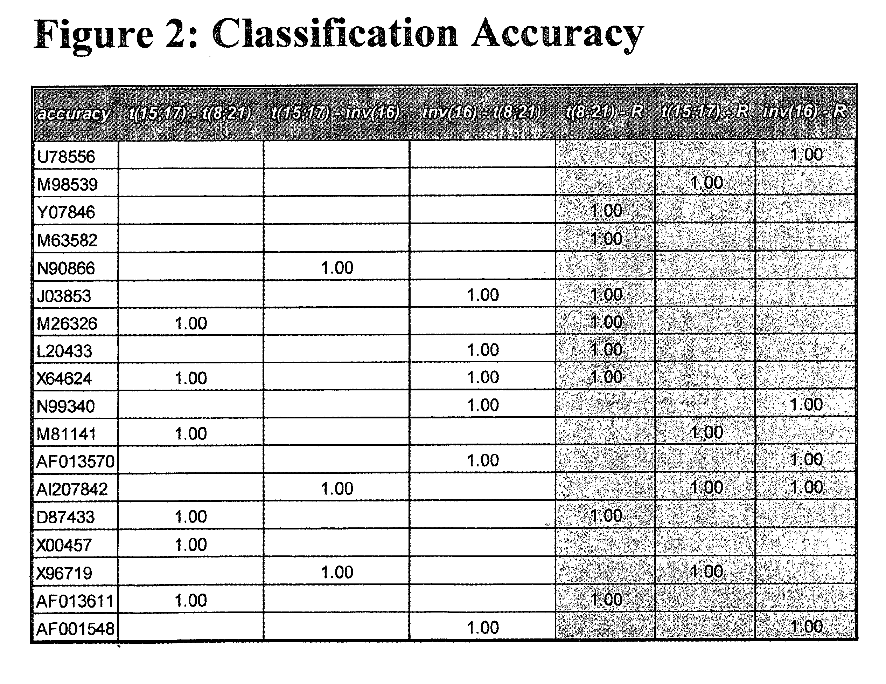

From this point of view it was found that a set of 17 genes was sufficient to distinguish distinct AML subtypes from each other with high precision (Tables 1). The classification model was able to identify the 4 morphologically and 3 cytogenetically and molecular biological different subtypes AML with t(8;21), with t(15;17),and with inv(16) (Figures 1a-b, 2).

In conclusion, by comparison of gene expression profiles of AML samples (3 tested genetic subtypes t(8;21), t(15;17) and inv(16)) genes could be identified which allowed a differentiation between each individual AML subtype in detail. It could be shown for the first time that these distinct abnormalities on the genomic level relate to a specific gene expression pattern. In other words, in the experimental setting the knowledge of the expression status of these designated genes was sufficient to predict the genetic abnormality and allows the diagnosis of specific genetically defined subtypes of AML (Table 1).

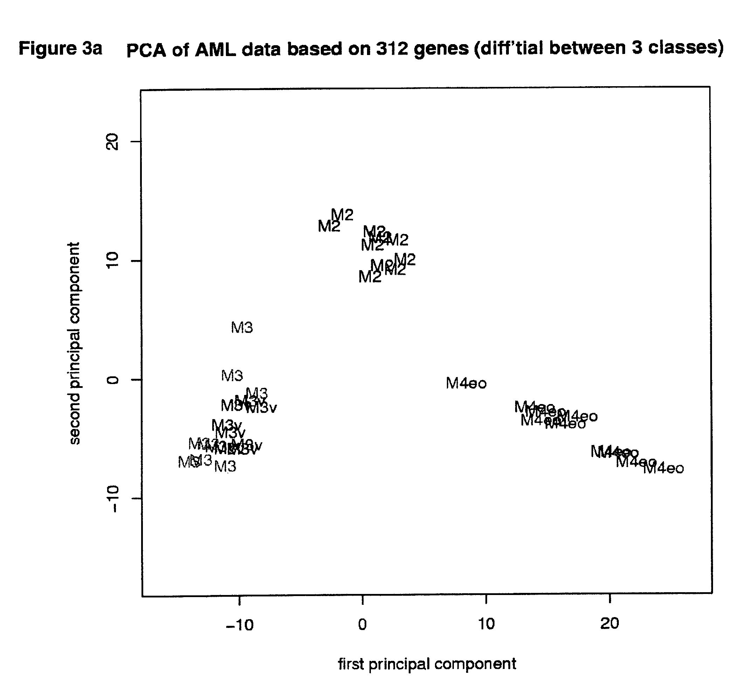

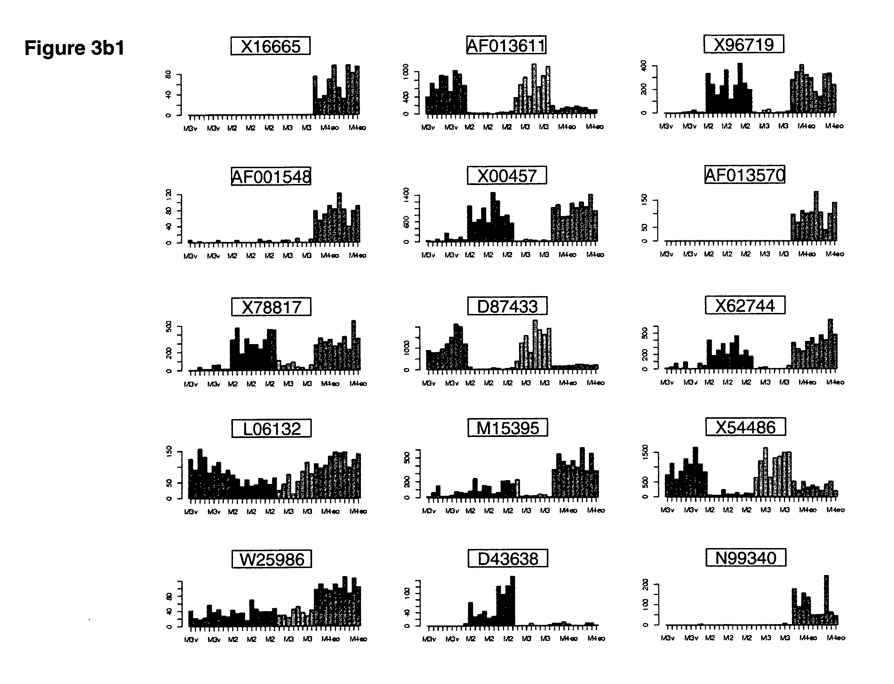

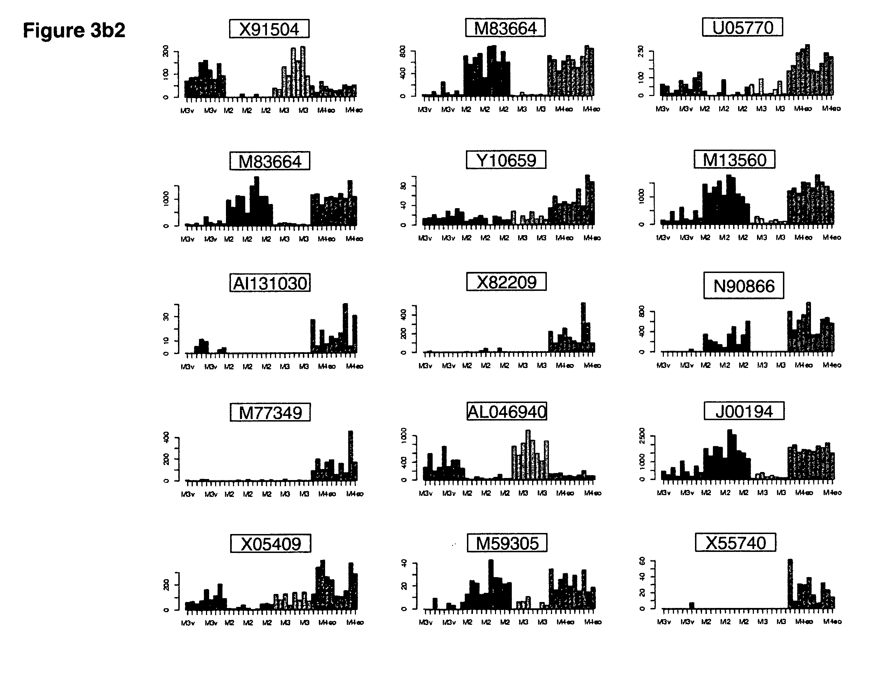

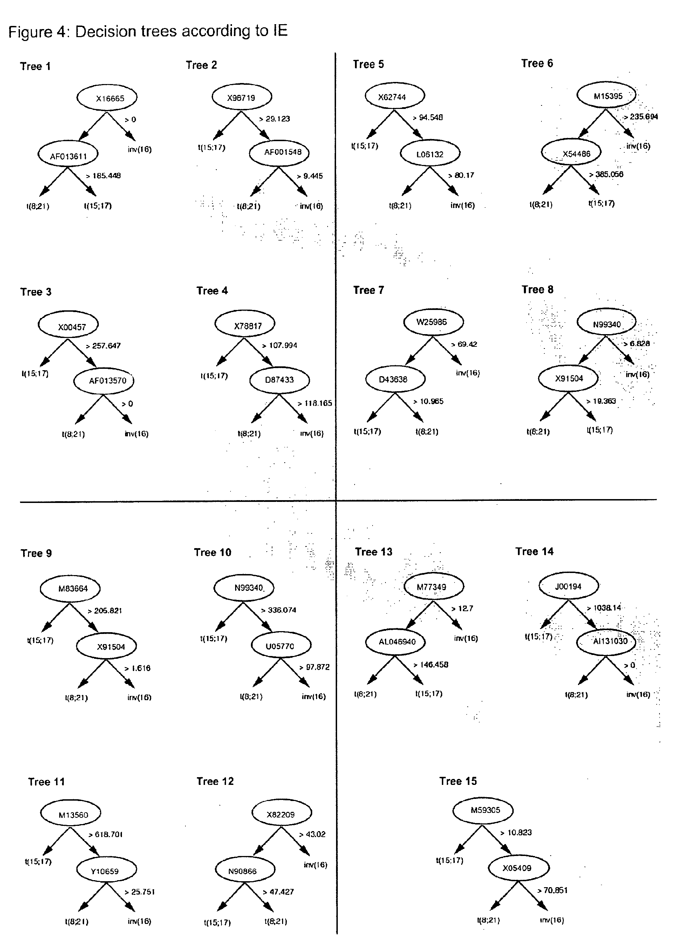

Results of methods described in I(E) are shown in Table 2 and Figures 3a+b, 1/2 and 4.

(II) Pair-wise comparisons between normal bone marrow, AML, ALL, CML, and CLL

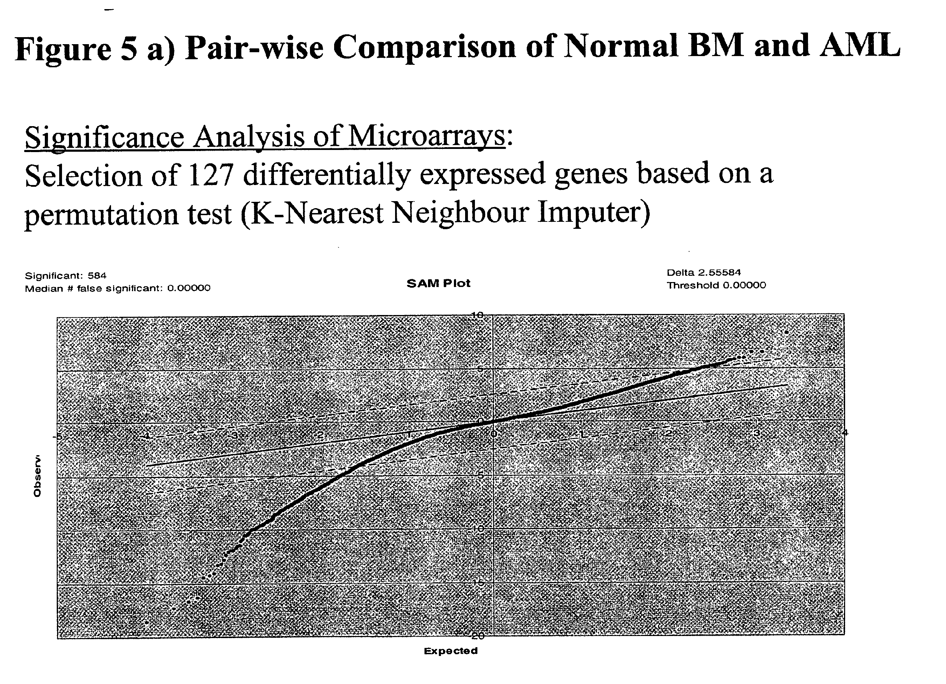

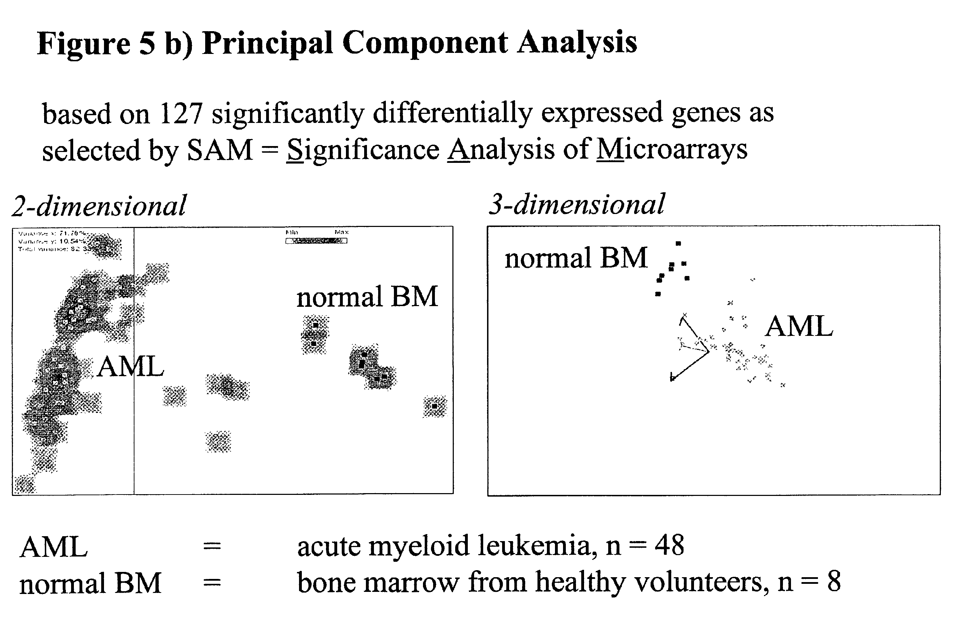

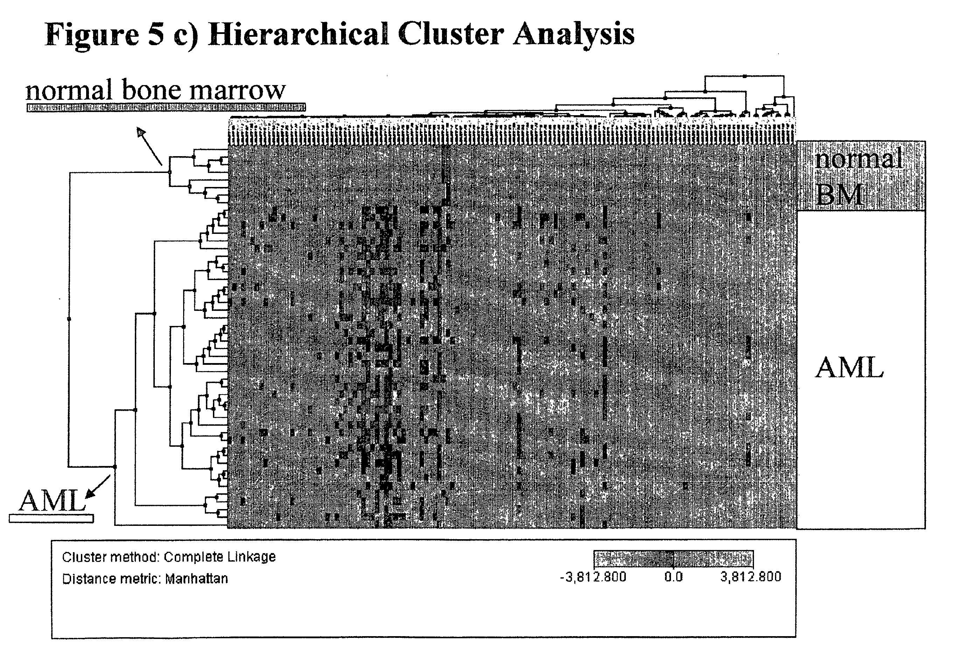

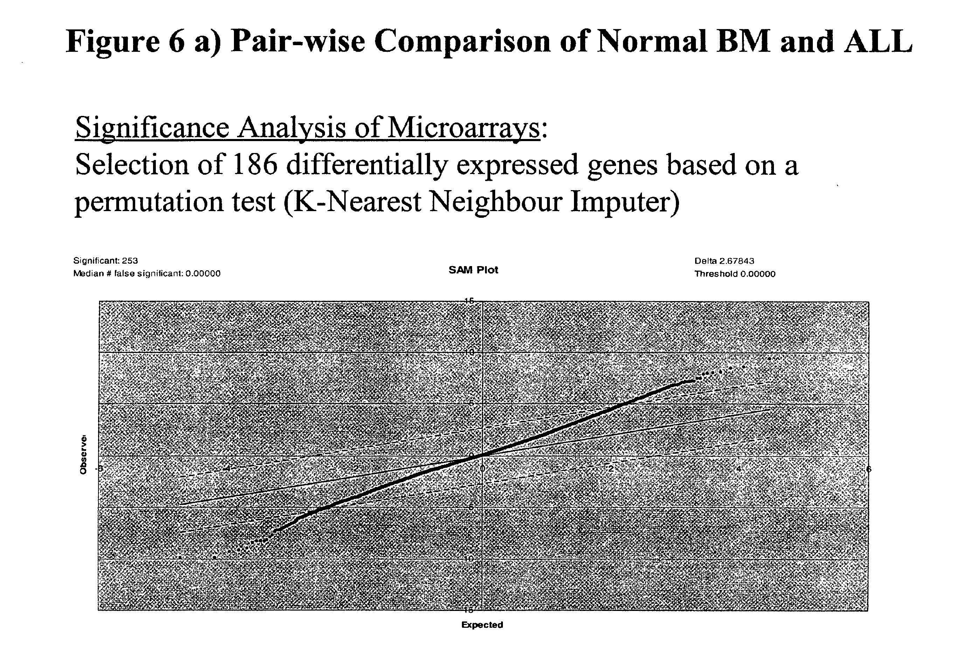

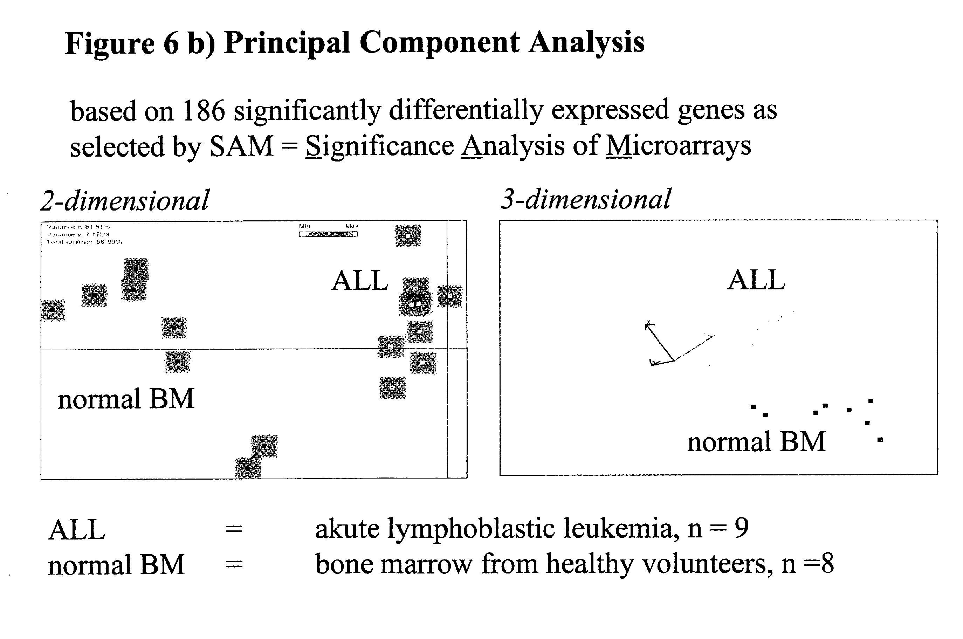

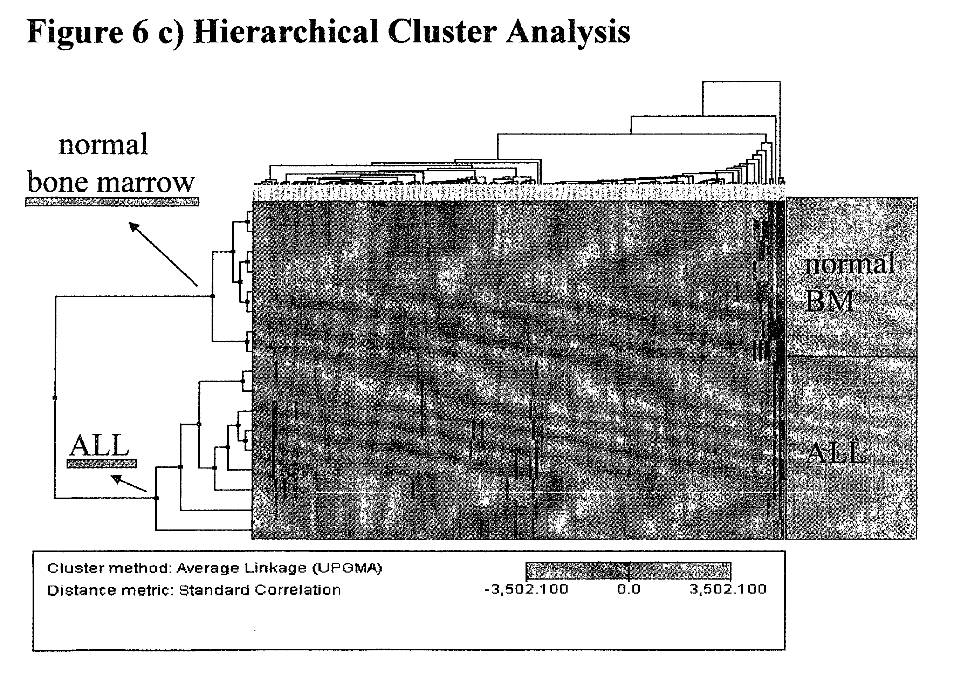

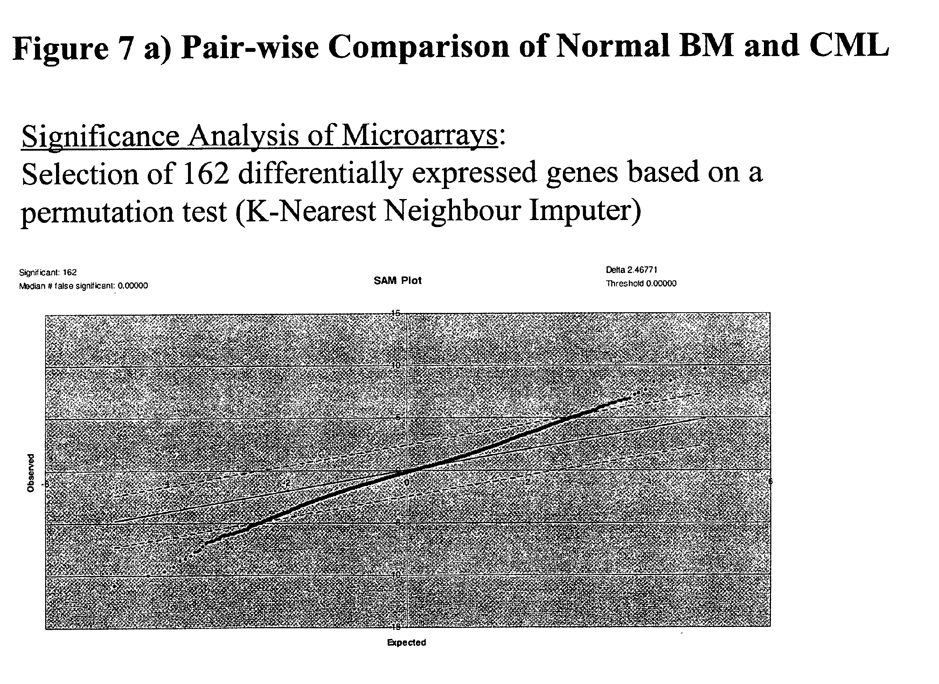

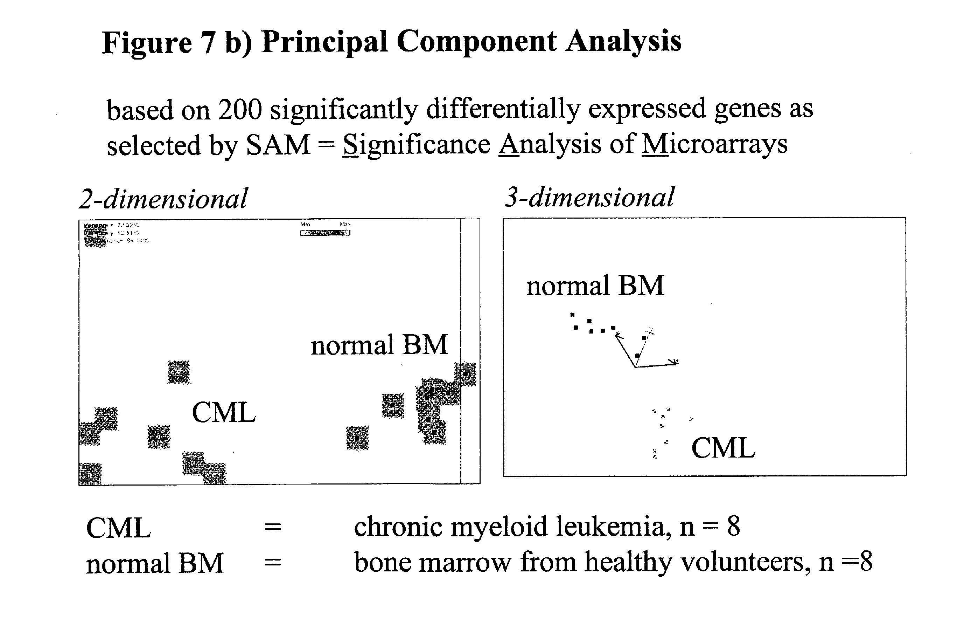

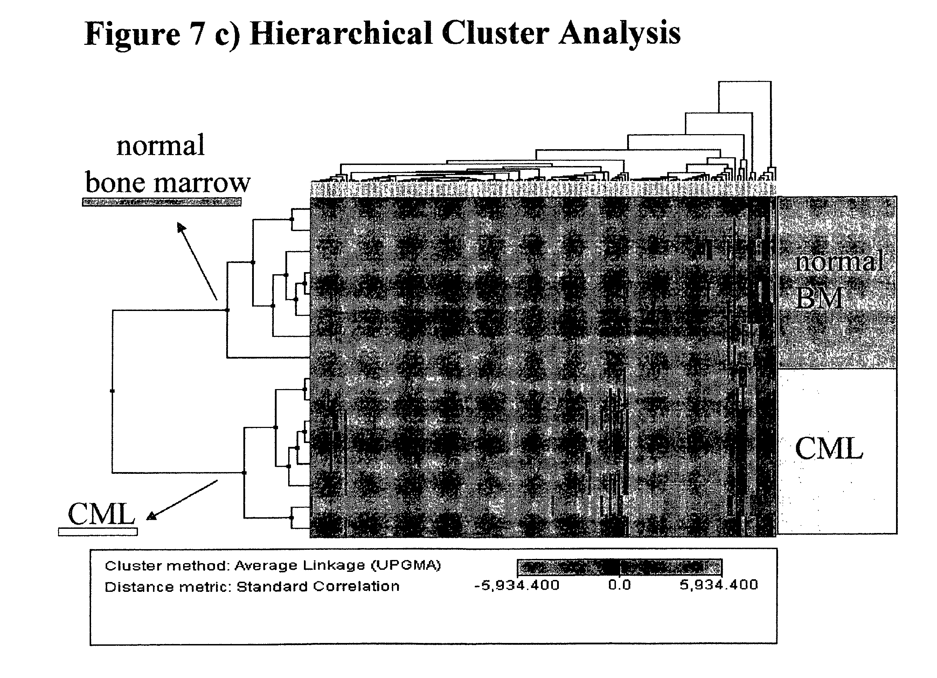

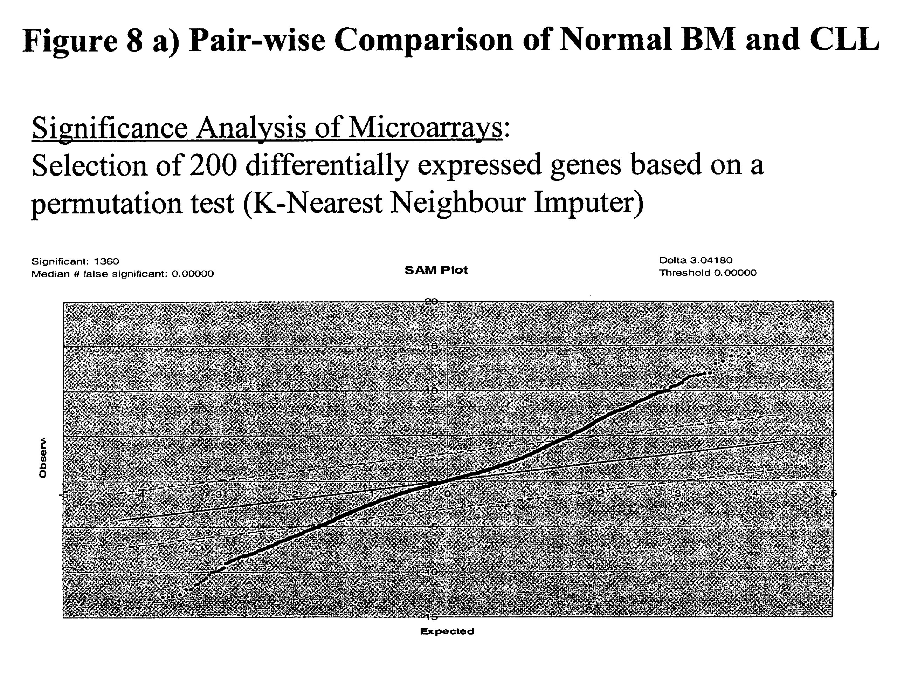

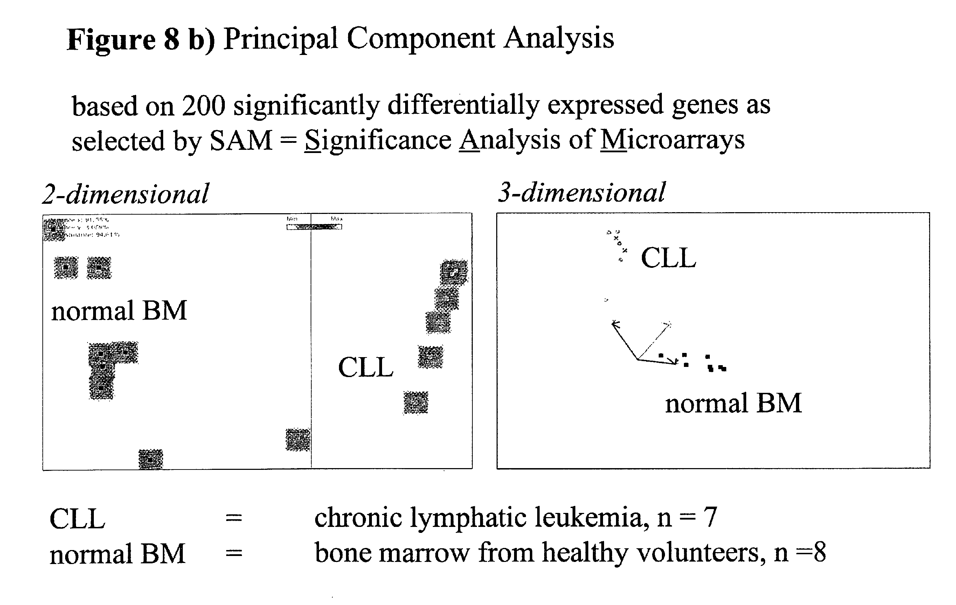

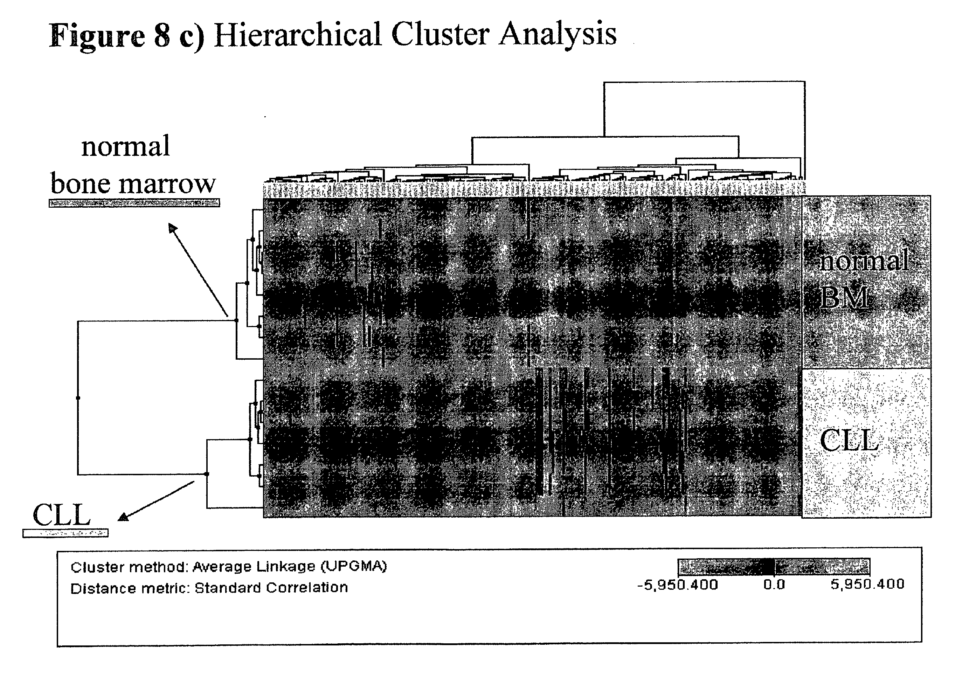

By pair-wise comparisons gene expression profiles of 8 cases of normal bone marrow, 48 AML, 9 ALL, 8 CML, and 7 CLL were evaluated. These led to the identification of subtype-specific genes (Tables 3-12; Figures 5a-c, 6a-c, 7a-c, 8a-c).

(III) AML classified according to WHO proposal

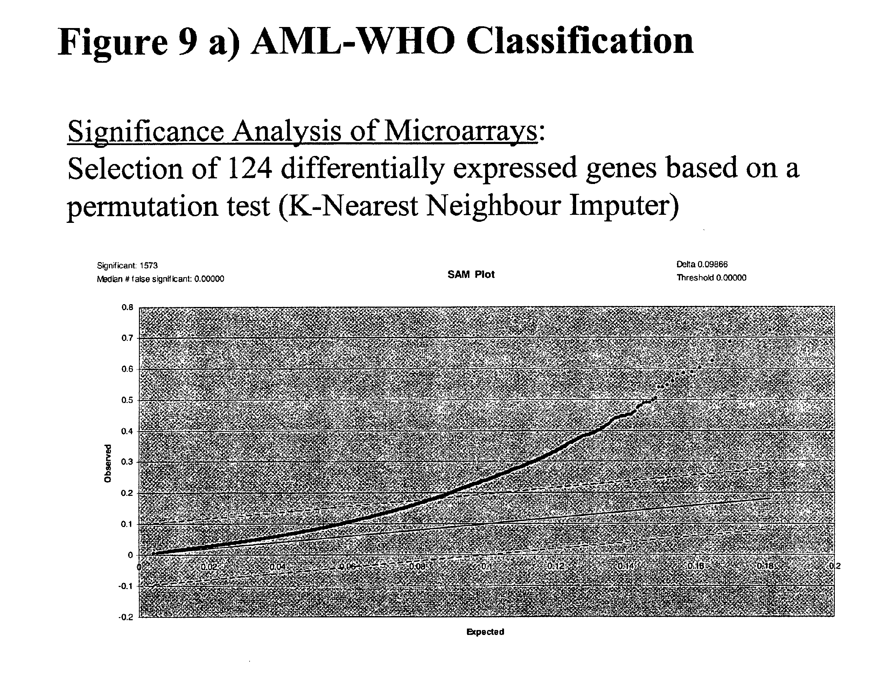

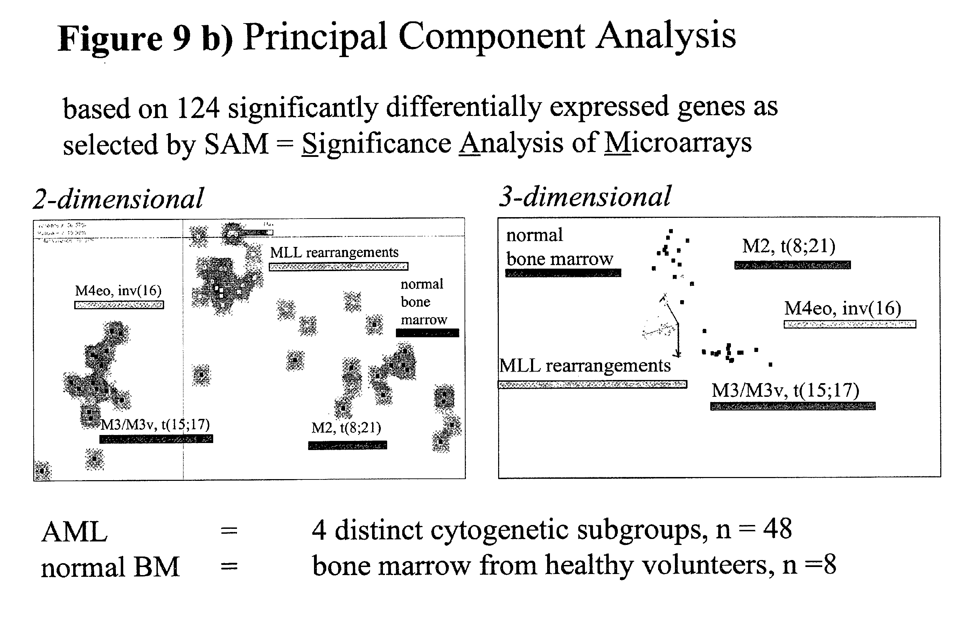

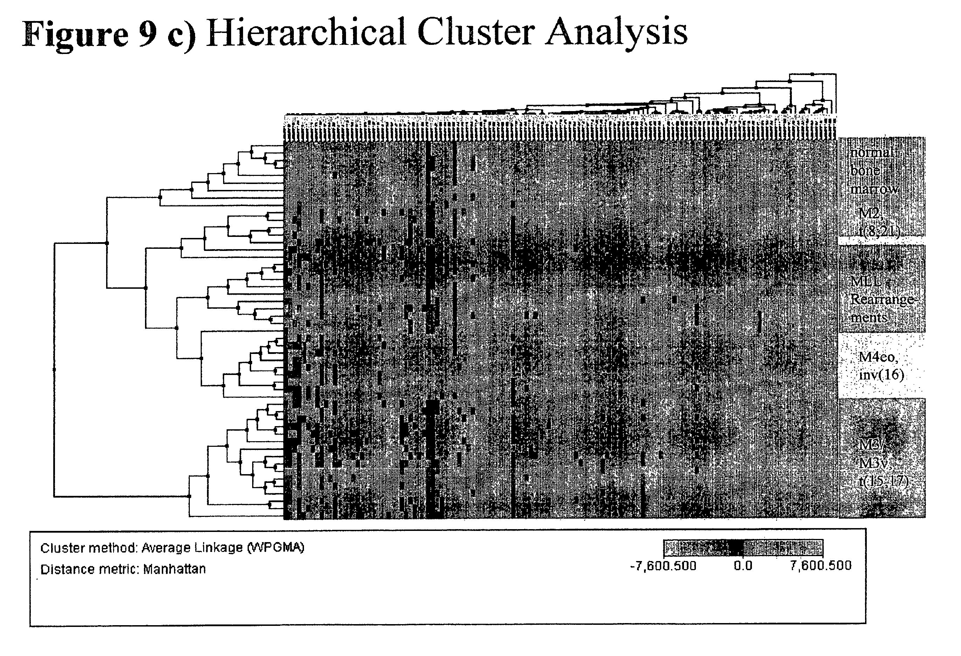

To allow classification of AML subtypes according to the new WHO proposal the gene expression profiles of four genetically defined AML subtypes (t(8;21) n= 9; t(15;17) n= 18; inv(16) n= 10; 11q23/MLL aberrations n= 11) was used. This led to the identification of subtype-specific genes (Table 13, Figures 9a-c) .

(IV) Normal bone marrow *versus* distinct genetic subtypes of AML

The gene expression profiles of normal bone marrow (n= 8) and of four genetically defined AML subtypes (t (8;21) n= 9; t(15;17) n= 18; inv(16) n= 10; 11q23/MLL aberrations n= 10) was used. This led to the identification of genes that allow the distinction between normal bone marrow and each of the four AML subtypes (Table 14).

(V) Identification of genes specifically separating normal bone marrow, AML, ALL, CML, and CLL

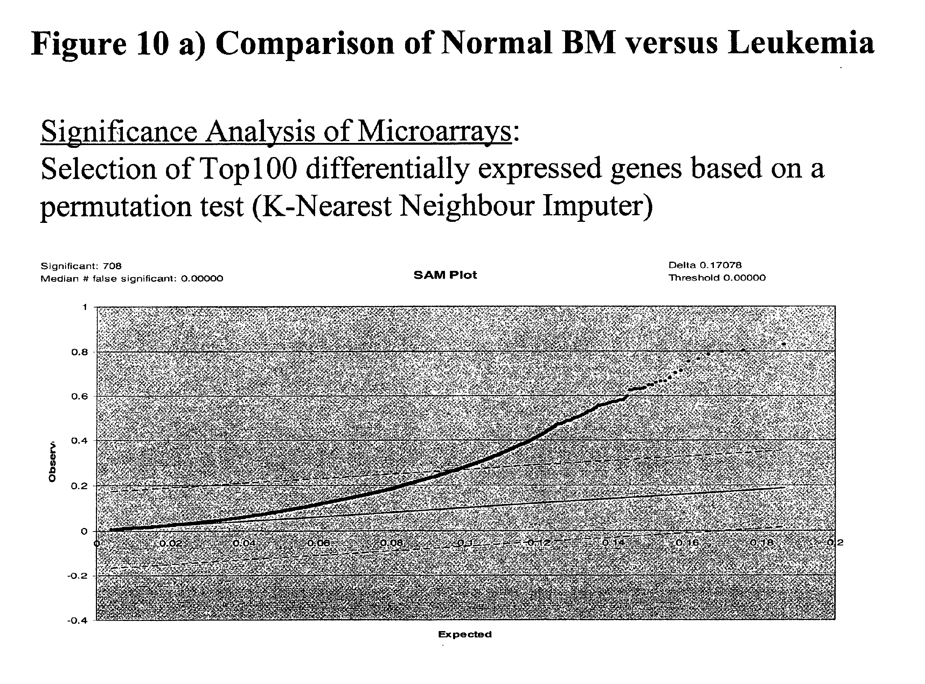

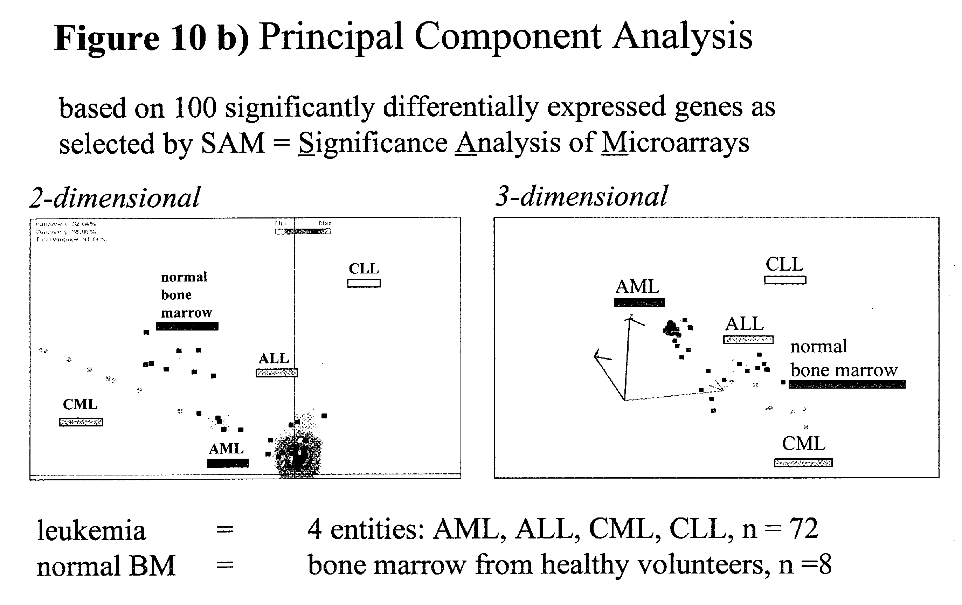

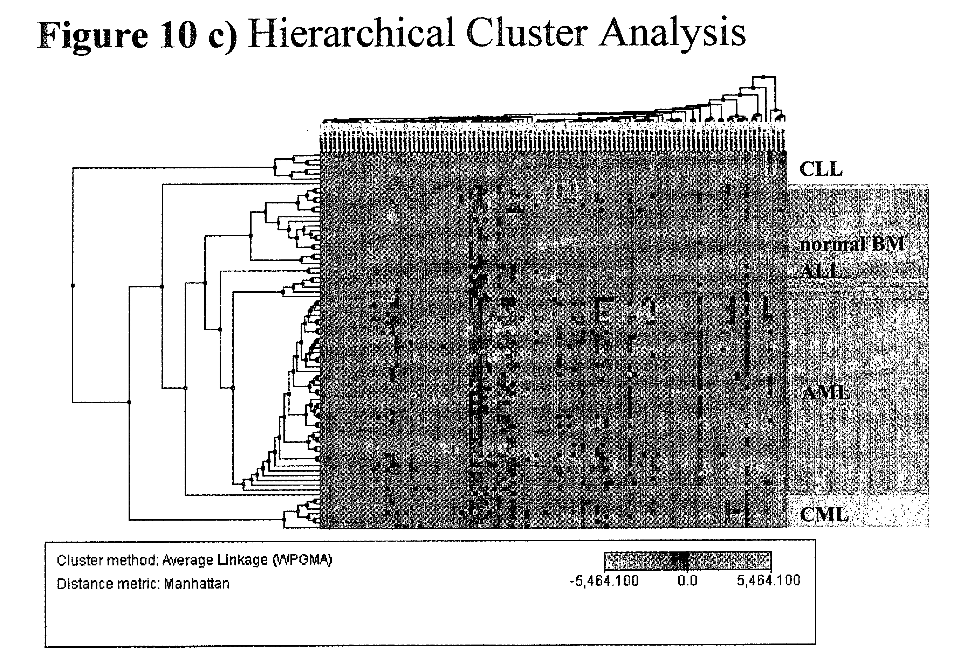

The gene expression profiles of normal bone marrow (n= 8) and of AML (n= 48), ALL (n= 9), CML (n= 8), and CLL (n= 7) was used. This led to the identification of genes that allow the distinction between normal bone marrow and each of the four leukemia subtypes (Table 15, Figures 10a-c).

**Claims**

1.   A diagnostic composition comprising at least one nucleic acid molecule which is capable of specifically hybridizing to the mRNA of at least one gene listed in Table 1 - 15.

2. The diagnostic composition of claim 1 comprising at least 20 nucleic acid molecules which are capable of specifically hybridizing to the mRNAs of at least 20 genes listed in Table 1 - 15.

3. The diagnostic composition of claim 2 comprising at least 40 nucleic acid molecules which are capable of specifically hybridizing to the mRNAs of at least 40 genes listed in Table 1 - 15.

4. The diagnostic composition of any one of claims 1 to 3, wherein the nucleic acid molecules are bound to a solid support or to non-immobilised particles in solution.

5. The diagnostic composition of claim 4, wherein the solid support is a microarray.

6. The diagnostic composition of any one of claims 1 to 5, wherein the nucleic acid molecule has a length of at least 13 nucleotides.

7. Use of (a) nucleic acid molecule(s) as defined in any one of claims 1 to 6 for the preparation of a diagnostic composition for the diagnosis of a leukemia or a disposition to a leukemia.

8. Use according to claim 7, wherein the leukemia is acute myeloid leukemia.

9. Use of (a) nucleic acid molecule(s) as defined in any one of claims 1 to 6 for the preparation of a diagnostic composition for the diagnosis of a leukemia-subtype.

10. Use according to claim 9, wherein the diagnosis is AML, CML, ALL or CLL.

11. Use according to claim 10, wherein the FAB-subtypes are AML M2, AML M3, AML M3v, AML M4eo and/or AML with 11q23/MLL aberations.

# Figure 1a: Principal Component Analysis

FAB-subtype M2 with t(8;21)

FAB-subtype M3/M3v
with t(15;17)

FAB-subtype M4eo
with inv(16)

# Figure 1b: Hierarchical Cluster Analysis

Cluster method: single linkage

Distance metric: euclidean

FAB-subtype M3/M3v with t(15;17)

FAB-subtype M2 with t(8;21)

FAB-subtype M4eo with inv(16)

EP 1 308 522 A1

# Figure 2: Classification Accuracy

| accuracy | t(15;17) - t(8;21) | t(15;17) - inv(16) | inv(16) - t(8;21) | t(8;21) - R | t(15;17) - R | inv(16) - R |
|---|---|---|---|---|---|---|
| U78556 | | | | | | 1.00 |
| M98539 | | | | | 1.00 | |
| Y07846 | | | | 1.00 | | |
| M63582 | | | | 1.00 | | |
| N90866 | | 1.00 | | | | |
| J03853 | | | 1.00 | 1.00 | | |
| M26326 | 1.00 | | | 1.00 | | |
| L20433 | | | 1.00 | 1.00 | | |
| X64624 | 1.00 | | 1.00 | 1.00 | | |
| N99340 | | | 1.00 | | | 1.00 |
| M81141 | 1.00 | | | | 1.00 | |
| AF013570 | | | 1.00 | | | 1.00 |
| AI207842 | | 1.00 | | | 1.00 | 1.00 |
| D87433 | 1.00 | | | 1.00 | | |
| X00457 | 1.00 | | | | | |
| X96719 | | 1.00 | | | 1.00 | |
| AF013611 | 1.00 | | | 1.00 | | |
| AF001548 | | | 1.00 | | | 1.00 |

EP 1 308 522 A1

Figure 3a    PCA of AML data based on 312 genes (diff'tial between 3 classes)

**Figure 3b1**

Figure 3b2

U05770 · M83664 · X91504 · M13560 · Y10659 · M83664 · N90866 · X82209 · AI131030 · J00194 · AL046940 · M77349 · X55740 · M59305 · X05409

Figure 4: Decision trees according to IE

# Figure 5 a) Pair-wise Comparison of Normal BM and AML

## Significance Analysis of Microarrays:
Selection of 127 differentially expressed genes based on a permutation test (K-Nearest Neighbour Imputer)

Significant: 584
Median # false significant: 0.00000

**SAM Plot**

Delta 2.55584
Threshold 0.00000

Observ

Expected

EP 1 308 522 A1

# Figure 5 b) Principal Component Analysis

based on 127 significantly differentially expressed genes as
selected by SAM = $\underline{S}$ignificance $\underline{A}$nalysis of $\underline{M}$icroarrays

*2-dimensional*

*3-dimensional*

EP 1 308 522 A1

| AML | = | acute myeloid leukemia, n = 48 |
| normal BM | = | bone marrow from healthy volunteers, n = 8 |

# Figure 5 c) Hierarchical Cluster Analysis

normal bone marrow

normal
BM

AML

AML

Cluster method: Complete Linkage
Distance metric: Manhattan

-3,812.800    0.0    3,812.800

# Figure 6 a) Pair-wise Comparison of Normal BM and ALL

<u>Significance Analysis of Microarrays</u>:
Selection of 186 differentially expressed genes based on a
permutation test (K-Nearest Neighbour Imputer)

Significant: 253
Median # false significant: 0.00000

**SAM Plot**

Delta 2.67843
Threshold 0.00000

Observe

Expected

# Figure 6 b) Principal Component Analysis

based on 186 significantly differentially expressed genes as
selected by SAM = Significance Analysis of Microarrays

*2-dimensional*

*3-dimensional*

| | | |
|---|---|---|
| ALL | = | akute lymphoblastic leukemia, n = 9 |
| normal BM | = | bone marrow from healthy volunteers, n =8 |

# Figure 6 c) Hierarchical Cluster Analysis

normal
bone marrow

normal
BM

ALL

ALL

Cluster method: Average Linkage (UPGMA)
Distance metric: Standard Correlation

-3,502.100    0.0    3,502.100

# Figure 7 a) Pair-wise Comparison of Normal BM and CML

<u>Significance Analysis of Microarrays</u>:
Selection of 162 differentially expressed genes based on a
permutation test (K-Nearest Neighbour Imputer)

Significant: 162
Median # false significant: 0.00000

SAM Plot

Delta 2.46771
Threshold 0.00000

Observed

Expected

# Figure 7 b) Principal Component Analysis

based on 200 significantly differentially expressed genes as selected by SAM = Significance Analysis of Microarrays

*2-dimensional*

normal BM

CML

*3-dimensional*

normal BM

CML

| | | |
|---|---|---|
| CML | = | chronic myeloid leukemia, n = 8 |
| normal BM | = | bone marrow from healthy volunteers, n = 8 |

EP 1 308 522 A1

# Figure 7 c) Hierarchical Cluster Analysis

normal
bone marrow

CML

normal
BM

CML

Cluster method: Average Linkage (UPGMA)
Distance metric: Standard Correlation

-5,934.400    0.0    5,934.400

EP 1 308 522 A1

# Figure 8 a) Pair-wise Comparison of Normal BM and CLL

<u>Significance Analysis of Microarrays</u>:
Selection of 200 differentially expressed genes based on a
permutation test (K-Nearest Neighbour Imputer)

Significant: 1360
Median # false significant: 0.00000

**SAM Plot**

Delta 3.04180
Threshold 0.00000

Observe

Expected

# Figure 8 b) Principal Component Analysis

based on 200 significantly differentially expressed genes as selected by SAM = Significance Analysis of Microarrays

*2-dimensional*

*3-dimensional*

CLL        =        chronic lymphatic leukemia, n = 7

normal BM   =        bone marrow from healthy volunteers, n =8

EP 1 308 522 A1

# Figure 8 c) Hierarchical Cluster Analysis

normal bone marrow

normal BM

CLL

CLL

Cluster method: Average Linkage (UPGMA)
Distance metric: Standard Correlation

-5,950.400    0.0    5,950.400

EP 1 308 522 A1

# Figure 9 a) AML-WHO Classification

<u>Significance Analysis of Microarrays</u>:
Selection of 124 differentially expressed genes based on a permutation test (K-Nearest Neighbour Imputer)

Significant: 1573
Median # false significant: 0.00000

**SAM Plot**

Delta 0.09866
Threshold 0.00000

EP 1 308 522 A1

# Figure 9 b) Principal Component Analysis

based on 124 significantly differentially expressed genes as
selected by SAM = Significance Analysis of Microarrays

*2-dimensional*

*3-dimensional*

AML       =       4 distinct cytogenetic subgroups, n = 48

normal BM    =       bone marrow from healthy volunteers, n = 8

# Figure 9 c) Hierarchical Cluster Analysis

Cluster method: Average Linkage (WPGMA)

Distance metric: Manhattan

-7,600.500  0.0  7,600.500

normal bone marrow

M2, t(8;21)

MLL Rearrangements

M4eo, inv(16)

M3/M3v, t(15;17)

EP 1 308 522 A1

# Figure 10 a) Comparison of Normal BM versus Leukemia

Significance Analysis of Microarrays:
Selection of Top100 differentially expressed genes based on a
permutation test (K-Nearest Neighbour Imputer)

Significant: 708
Median # false significant: 0.00000

**SAM Plot**

Delta 0.17078
Threshold 0.00000

# Figure 10 b) Principal Component Analysis

based on 100 significantly differentially expressed genes as
selected by SAM = Significance Analysis of Microarrays

*2-dimensional*

*3-dimensional*

leukemia = 4 entities: AML, ALL, CML, CLL, n = 72

normal BM = bone marrow from healthy volunteers, n = 8

EP 1 308 522 A1

Figure 10 c) Hierarchical Cluster Analysis

CLL

normal BM
ALL

AML

CML

Cluster method: Average Linkage (WPGMA)
Distance metric: Manhattan

-5,464.100    0.0    5,464.100

**European Patent Office** **PARTIAL EUROPEAN SEARCH REPORT** **Application Number**

which under Rule 45 of the European Patent Convention EP 01 12 6244
shall be considered, for the purposes of subsequent
proceedings, as the European search report

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 01 47944 A (CURAGEN CORP ;LEACH MARTIN (US); SHIMKETS RICHARD A (US)) 5 July 2001 (2001-07-05) See SNP oligonucleotide #4477, see claims 41-44 --- | 1-11 | C12Q1/68 |
| X | "Human Genome U95Av2" AFFYMETRIX PRODUCT INFORMATION, [Online] XP002215481 Retrieved from the Internet: <URL:http://www.affymetrix.com> [retrieved on 2002-10-02] * the whole document * --- | 1-11 | |
| X | ZUCMAN-ROSSI J ET AL: "Identification of new members of the Gas2 and Ras families in the 22q12 chromosome region." GENOMICS. UNITED STATES 15 DEC 1996, vol. 38, no. 3, 15 December 1996 (1996-12-15), pages 247-254, XP002215482 ISSN: 0888-7543 | 1,6-11 | |
| Y | * the whole document * --- -/-- | 2-5 | C12Q |

**TECHNICAL FIELDS SEARCHED (Int.Cl.7)**

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| THE HAGUE | 8 October 2002 | Hagenmaier, S |

EPO FORM 1503 03.82 (P04C07)

| European Patent Office | INCOMPLETE SEARCH SHEET C | Application Number EP 01 12 6244 |
|---|---|---|

Claim(s) not searched:
        1-11 (all partially)

Reason for the limitation of the search:

The compositions and methods of the present claims 1-11 relate to an extremely large number of possible sets of genes . In fact, the claims contain so many possible permutations that a lack of clarity (and conciseness) within the meaning of Article 84 EPC arises to such an extent as to render a meaningful search of the claims impossible. Consequently, the search has to be limited to compositions and methods relating to a specific gene listed in one of the tables 1-15 as such (see also sheet B).

**European Patent** **PARTIAL EUROPEAN SEARCH REPORT** **Application Number**

**Office**

EP 01 12 6244

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| D,Y | GOLUB T R ET AL: "Molecular classification of cancer: Class discovery and class prediction by gene expression monitoring" SCIENCE, AMERICAN ASSOCIATION FOR THE ADVANCEMENT OF SCIENCE,, US, vol. 286, no. 5439, 15 October 1999 (1999-10-15), pages 531-537, XP002207658 ISSN: 0036-8075 * the whole document * --- | 1-11 | |
| Y | EP 1 043 676 A (WHITEHEAD BIOMEDICAL INST) 11 October 2000 (2000-10-11) * the whole document * --- | 1-11 | TECHNICAL FIELDS SEARCHED (Int.Cl.7) |
| Y | ALIZADEH A A ET AL: "DISTINCT TYPES OF DIFFUSE LARGE B-CELL LYMPHOMA IDENTIFIED BY GENE EXPRESSION PROFILING" NATURE, MACMILLAN JOURNALS LTD. LONDON, GB, vol. 403, 3 February 2000 (2000-02-03), pages 503-512, XP002943414 ISSN: 0028-0836 * the whole document * --- | 1-11 | |
| Y | ALIZADEH A ET AL: "THE LYMPHOCHIP: A SPECIALIZED CDNA MICROARRAY FOR THE GENOMIC-SCALE ANALYSIS OF GENE EXPRESSION IN NORMAL AND MALIGNANT LYMPHOCYTES" COLD SPRING HARBOR SYMPOSIA ON QUANTITATIVE BIOLOGY, BIOLOGICAL LABORATORY, COLD SPRING HARBOR, NY, US, vol. 64, no. 1, 1999, pages 71-78, XP001099007 ISSN: 0091-7451 * the whole document * --- | 1-11 | |

-/--

EP 1 308 522 A1

**PARTIAL EUROPEAN SEARCH REPORT**

Application Number

EP 01 12 6244

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| Y | VIRTANEVA K ET AL: "EXPRESSION PROFILING REVEALS FUNDAMENTAL BIOLOGICAL DIFFERENCES IN ACUTE MYELOID LEUKEMIA WITH ISOLATED TRISOMY 8 AND NORMAL CYTOGENETICS" PROCEEDINGS OF THE NATIONAL ACADEMY OF SCIENCES OF USA, NATIONAL ACADEMY OF SCIENCE. WASHINGTON, US, vol. 98, no. 3, 30 January 2001 (2001-01-30), pages 1124-1129, XP002952627 ISSN: 0027-8424 * the whole document * | 1-11 | |
| A | MIYAZATO A ET AL: "IDENTIFICATION OF MYELODYSPLASTIC SYNDROME-SPECIFIC GENES BY DNA MICROARRAY ANALYSIS WITH PURIFIED HEMATOPOIETIC STEM CELL FRACTION" BLOOD, W.B.SAUNDERS COMPAGNY, ORLANDO, FL, US, vol. 98, no. 2, 15 July 2001 (2001-07-15), pages 422-427, XP002952629 ISSN: 0006-4971 * the whole document * | | TECHNICAL FIELDS SEARCHED (Int.Cl.7) |
| A | ROSS D T ET AL: "SYSTEMATIC VARIATION IN GENE EXPRESSION PATTERNS IN HUMAN CANCER CELL CEL LINES" NATURE GENETICS, NATURE AMERICA, NEW YORK, US, vol. 24, March 2000 (2000-03), pages 227-235, XP002933374 ISSN: 1061-4036 * the whole document * | | |

-/--

EPO FORM 1503 03.82 (P04C10)

| European Patent Office | PARTIAL EUROPEAN SEARCH REPORT | Application Number EP 01 12 6244 |
|---|---|---|

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | SORLIE T ET AL: "Gene expression patterns of breast carcinomas distinguish tumor subclasses with clinical implications." PROCEEDINGS OF THE NATIONAL ACADEMY OF SCIENCES OF THE UNITED STATES OF AMERICA. UNITED STATES 11 SEP 2001, vol. 98, no. 19, 11 September 2001 (2001-09-11), pages 10869-10874, XP002215483 ISSN: 0027-8424 * the whole document * --- | | |
| T | SCHOCH CLAUDIA ET AL: "Acute myeloid leukemias with reciprocal rearrangements can be distinguished by specific gene expression profiles." PROCEEDINGS OF THE NATIONAL ACADEMY OF SCIENCES OF THE UNITED STATES OF AMERICA. UNITED STATES 23 JUL 2002, vol. 99, no. 15, 23 July 2002 (2002-07-23), pages 10008-10013, XP002215484 ISSN: 0027-8424 * the whole document * --- | 1-11 | TECHNICAL FIELDS SEARCHED (Int.Cl.7) |
| T | WO 02 24956 A (DANA FARBER CANCER INST INC ;RAMASWAMY SRIDHAR (US); WHITEHEAD BIO) 28 March 2002 (2002-03-28) * the whole document * ----- | 1-11 | |

EPO FORM 1503 03.82 (P04C10)

**EP 1 308 522 A1**

European Patent
Office

---

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

---

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-11 (all partially)

| European Patent Office | **LACK OF UNITY OF INVENTION** **SHEET B** | Application Number EP 01 12 6244 |

The Search Division considers that the present European patent application does not comply with the requirements of unity of invention and relates to several inventions or groups of inventions, namely:

1. Claims: 1-11 (all partially)

   Invention 1:

   Diagnostic composition comprising at least one nucleic acid molecule which is capable of specifically hybridizing to the mRNA of GAR22 and the use of such a nucleic acid for the preparation of a diagnostic composition for the diagnosis of leukemia.

2. Claims: 1-11 (all partially)

   Inventions 2-1700:

   Diagnostic composition comprising at least one nucleic acid molecule which is capable of specifically hybridizing to the mRNA of HLA-DPA1 and the use of such a nucleic acid for the preparation of a diagnostic composition for the diagnosis of leukemia.

   ..ibidem for each gene listed in table 1-15

**ANNEX TO THE EUROPEAN SEARCH REPORT
ON EUROPEAN PATENT APPLICATION NO.**                 EP 01 12 6244

This annex lists the patent family members relating to the patent documents cited in the above-mentioned European search report.
The members are as contained in the European Patent Office EDP file on
The European Patent Office is in no way liable for these particulars which are merely given for the purpose of information.

08-10-2002

| Patent document cited in search report | | Publication date | Patent family member(s) | | Publication date |
|---|---|---|---|---|---|
| WO 0147944 | A | 05-07-2001 | AU<br>EP<br>WO | 2914501 A<br>1244688 A1<br>0147944 A2 | 09-07-2001<br>02-10-2002<br>05-07-2001 |
| EP 1043676 | A | 11-10-2000 | CA<br>EP<br>JP | 2304876 A1<br>1043676 A2<br>2001017171 A | 09-10-2000<br>11-10-2000<br>23-01-2001 |
| WO 0224956 | A | 28-03-2002 | AU<br>WO<br>US | 9280201 A<br>0224956 A2<br>2002110820 A1 | 02-04-2002<br>28-03-2002<br>15-08-2002 |

EPO FORM P0459

For more details about this annex : see Official Journal of the European Patent Office, No. 12/82